# FASCICULE DE BREVET EUROPEEN

(11) **EP 1 062 223 B1**
(45) Date de publication et mention de la délivrance du brevet: **01.08.2007**
(21) Numéro de dépôt: 99908998.0
(22) Date de dépôt: 09.03.1999
(51) Int. Cl.: C07H 17/065, A61Q 19/00, C07H 15/203

(54) **COMPOSITION CONTENANT UN PRECURSEUR HYDROLYSABLE PAR LA GLUCOCEREBROSIDASE**
ZUSAMMENSETZUNG DIE EINEN DURCH GLUCOCEREBROSIDASE HYDROLYSIERBAREN VORLÄUFER ENTHALTEND
COMPOSITION CONTAINING A PRECURSOR CAPABLE OF BEING HYDROLYSED BY GLUCOCEREBROSIDASE

(30) Priorité: 10.03.1998 FR 9802888
(43) Date de publication de la demande: 27.12.2000
(73) Titulaire: Pierre Fabre Dermo-Cosmétique, 92100 Boulogne-Billancourt (FR)
(72) Inventeur: REDOULES, Daniel, F-31300 Toulouse (FR); TARROUX, Roger, F-31300 Toulouse (FR); PERIE, Jean-Jacques, F-31320 Castanet (FR)
(74) Mandataire: Warcoin, Jacques
(86) Numéro de dépôt international: PCT/FR1999/000521
(87) Numéro de publication internationale: WO 1999/046273

(56) Documents cités:
- EP-A- 0 027 987
- EP-A- 0 169 716
- EP-A- 0 487 404
- EP-A- 0 667 145
- WO-A-98/50005
- FR-A- 2 577 805
- W.M.HOLLERAN: "Permeability Barrier Requirements Regulate Epidermal B-Glucocerebrosidase." JOURNAL OF LIPID RESEARCH, vol. 35, 1994, pages 905-912, XP002089615 cité dans la demande
- M.A.LAMPE ET AL.: "Human Epidermal Lipids : Characterization and Modulations During Differentiation." JOURNAL OF LIPID RESEARCH, vol. 24, 1983, pages 131-140, XP002089616 cité dans la demande

## Description

La présente invention se rapporte à une composition cosmétique ou pharmaceutique pour application cutanée, contenant un composé apte à libérer une substance biologiquement active au contact de la peau par action d'une enzyme de la surface cutanée : la glucocérébrosidase.

On entend par «précurseur d'actif» un composé conjugué d'une molécule inactive avec une substance biologiquement active. Un précurseur d'actif ne présente aucune activité biologique propre (ou une faible activité), et libère, une fois métabolisé, ladite substance active dans le stratum corneum. Un «dérivé d'actif» se distingue d'un «précurseur d'actif» en ce qu'il est doté d'une activité biologique propre.

L'utilisation de précurseurs d'actifs en cosmétologie est de pratique courante, en particulier pour améliorer la stabilité des actifs dans les compositions et/ou pour faciliter la pénétration des actifs dans l'épiderme.

Les précurseurs de vitamines sont à ce titre largement utilisés.

En effet, les vitamines sont difficiles à formuler en raison de leur grande instabilité à l'air et à la chaleur.

On a d'abord proposé des dérivés de vitamines présentant une structure proche de la molécule active et dont la stabilité serait améliorée. Ces dérivés se sont cependant révélés trop peu actifs.

On leur préfère des précurseurs de vitamines, comme les esters d'acide gras, les esters de glycérides, les phosphates ou les sulfates de vitamines.

Les esters d'acide gras d'actif, comme le palmitate de vitamine E (ou palmitate d'alpha-tocophérol), présentent l'avantage non seulement de stabiliser l'actif mais également d'améliorer la pénétration cutanée d'un actif hydrosoluble, en augmentant son affinité avec le stratum corneum lipophile.

La demande de brevet FR-2 715 565 décrit une composition contenant deux précurseurs qui libèrent un même actif selon deux cinétiques différentes par un mécanisme synergétique. Les précurseurs sont choisis parmi les phosphates, sulfates, palmitates, propionates, acétates, férulates, éthers, amides et dérivés osidiques d'actifs.

Les compositions données en exemple contiennent deux précurseurs de la vitamine C : la vitamine C phosphatée et la vitamine C glucosylée. Ces deux précurseurs interagissent avec deux enzymes différentes, une phosphatase et une glucosidase, pour libérer la vitamine C de façon synergétique.

La demande de brevet EP-487 404 décrit l'utilisation d'un précurseur glucosylé de vitamine C (ou acide ascorbique) pour préparer une composition dermatologique qui exerce une activité de vitamine C dans l'organisme. Cette demande décrit en particulier les acides alpha-glucosyl-L-ascorbiques, qui n'ont aucune activité propre et qui sont hydrolyses en glucose et en acide L-ascorbique par une enzyme cutanée.

Certains dérivés osidiques d'actifs, présentant eux-mêmes une activité biologique, ont déjà été décrits.

Dans le cadre d'une étude sur les tocophérols (EP-169 716), il a été démontré que les dérivés glucosylés du delta-tocophérol présentent une activité anti-allergique. Le brevet JP-60 56 994 décrit l'activité antioxydante du dl-alpha-tacophéryl-glucose, du dl-alpha-tocophéryl-galactose et du d-delta-tocophéryl-galactose. L'activité de ces dérivés glucosylés du tocophérol s'est révélée cependant insuffisante si bien que leur utilisation en tant que substance biologiquement active présente peu d'intérêt.

FR 2 577 805 décrit une composition de dépigmentation de la peau comprenant un glucoside d'hydroquinone, en particulier l'albertine.

La présente invention a pour objectif de fournir une composition pour application cutanée qui libère un actif selon une cinétique telle que la concentration de l'actif dans l'épiderme reste supérieure à la concentration biologiquement active, mais inférieure à la concentration pro-inflammatoire.

La Demanderesse a en effet démontré que des compositions cosmétiques classiques contenant un actif comme la vitamine E génèrent un effet pro-inflammatoire. Cet effet pro-inflammatoire est la conséquence d'une suractivité de l'actif à la concentration pratiquée. Ceci est d'autant plus problématique que la concentration pratiquée est nécessaire pour observer les effets biologiques recherchés.

Afin d'éviter toute surconcentration de l'actif dans les heures suivant l'application cutanée de la composition, la Demanderesse propose d'utiliser un précurseur d'actif susceptible d'être hydrolysé par une enzyme dont la cinétique répond aux deux critères exposés ci-dessus.

La Demanderesse a démontré que la glucocérébrosidase permet de résoudre le problème posé. .

On sait que l'inhibition de la glucocérébrosidase par traitement topique de bromocondutirol B époxyde entraîne des perturbations aiguës de la fonction barrière (Holleran W.M., Elias P.M., «Permeability barrier requirements regulate epidermal β-glucocerebrosidase», J. Lipid. Res., 1994, 35, 905-912).

Ces résultats démontrent que, d'une part, cette enzyme est accessible par voie topique et, d'autre part, qu'elle joue un rôle essentiel dans l'équilibre du système cutané.

De plus, la glucocérébrosidase présente, dans le stratum corneum, une activité importante : les substrats naturels de cette enzyme, les glucocérébrosides, sont hydrolysés en céramides lesquels représentent 40 % en poids des lipides du stratum corneum (Lampe M.A., Williams M.L. and Elias P.M., «Human epidermal lipids: characterization and modulation during differentiation», J. Lipid. Res., 24, 131-140).

La Demanderesse a démontré par des études cliniques réalisées sur vingt-deux patients que l'activité de la glucocérébrosidase varie peu en fonction de la profondeur de la couche cornée, varie peu en fonction des individus et de la saison. La glucocérébrosidase est donc une hydrolase d'activité importante et constante.

La Demanderesse a, en outre, démontré que la cinétique d'hydrolyse de la glucocérébrosidase permet d'envisager l'utilisation d'un précurseur d'actif qui libère l'actif à une vitesse à la fois suffisante pour garantir son activité et modérée pour éviter toute intolérance due à une surconcentration d'actif.

C'est pourquoi, la présente invention décrit une composition cosmétique ou pharmaceutique pour application cutanée contenant un composé apte à libérer une substance biologiquement active au contact de la peau par action d'une enzyme cutanée caractérisée en ce que l'enzyme est la glucocérébrosidase.

L'expression «composé apte à libérer une substance biologiquement active» s'entend d'un composé doté d'une structure proche d'un substrat naturel de ladite enzyme.

Le composé apte à libérer la substance biologiquement active est un composé conjugué de ladite substance avec un glucide tel que:
- ladite substance biologiquement active présente un reste phénol dont les deux carbones en alpha de la fonction phénol sont non substitués, et
- la liaison chimique entre ladite substance biologiquement active et ledit glucide est une fonction éther entre le glucide et la fonction phénol de la substance biologiquement active.

Afin de faciliter la pénétration du précurseur d'actif dans le stratum corneum, la substance biologiquement active présente avantageusement un certain caractère lipophile.

Le glucide est un ose en C3-C6, comme le glucose, le mannose, le galactose, de préférence le glucopyranose ou un de ses dérivés.

La substance biologiquement active peut être une substance thérapeutiquement active choisie parmi le tioxolone, le paracétamol, l'oxyphénylbutazone, l'isoxsuprine, l'étiléfrine, le p-pentyloxy-phénol, le delta-tocophérol, le tocol et les flavonoïdes.

La formule des composés énumérés est donnée ci-dessous.

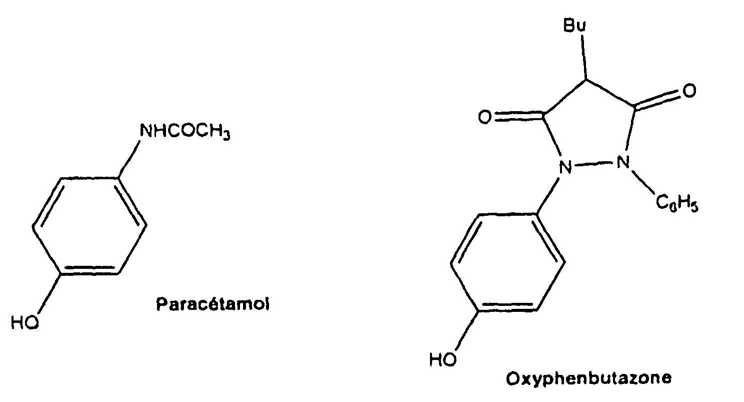

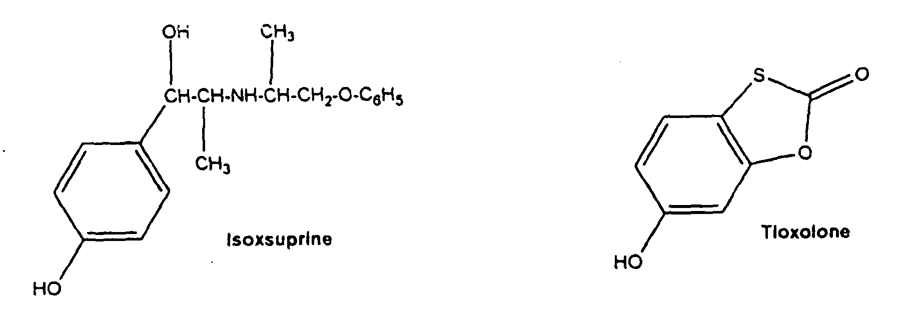

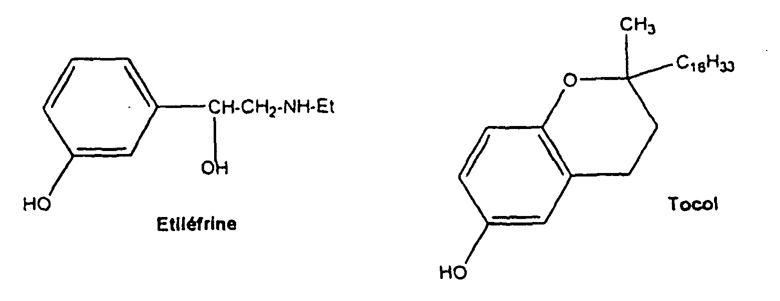

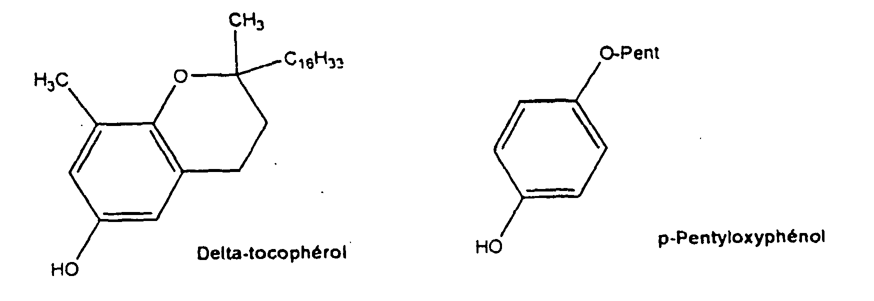

Le paracétamol correspond au p-acétamido-phénol. Dans les formules du tocol et du delta-tocophérol, C₁₆H₃₃ en position 2 du cycle 3,4-dihydrobenzopyrane est un substituant 4,8,12-triméthyltridécyle.

La substance biologiquement active peut avoir une action strictement au niveau de l'épiderme, notamment lorsqu'elle est choisie parmi le tocol, le delta-tocophérol, les flavonoïdes (qui sont des antioxydants) et la tioxolone (qui est un anti-séborrhéique).

La substance biologiquement active peut également avoir une action plus globale au niveau de l'épiderme ou à un autre niveau de l'organisme : le paracétamol est un analgésique, l'oxyphenbutazone un anti-inflammatoire, l'isoxsuprine un vasodilatateur, l'étiléfrine un hypotenseur et le p-pentyloxy-phénol un bactéricide.

La présente invention décrit également les composés conjugués d'une substance biologiquement active avec un glucide tels que :
- ladite substance biologiquement active, différente du delta-tocophérol, présente un reste phénol dont les deux carbones en alpha de la fonction phénol sont non substitués, et
- la liaison chimique entre ladite substance biologiquement active et ledit glucide est une fonction éther entre le glucide et la fonction phénol de la substance biologiquement active.

Les composés préférés dans le cadre de la présente invention sont tels que le glucide est un ose en C3-C6, notamment le glucose.

Les composés décrits sont choisis parmi le tioxonyl-glucopyranoside, l'oxyphenbutazonyl-glucopyranoside, le p-acétamidophényl-glucopyranoside, l'isoxsuprinyl-glucopyranoside, l'étiléfrinyl-glucopyranoside, le p-pentyloxyphényl-glucopyranoside et le tocol-glucopyranoside.

La présente invention a pour objet un composé conjugué, tel que défini dans la revendication 4, ainsi qu'une composition cosmétique ou pharmaceutique le contenant, telle que définie dans la revendication 6.

Les compositions décrites dans la présente invention contenant un composé apte à libérer le tocol, le delta-tocophérol ou un flavonoïde sont efficaces contre le vieillissement cutané, contre le dessèchement de la peau ou pour apaiser les effets dus aux expositions solaires et polluantes, ou contre la production de radicaux libres responsables du stress oxydatif cutané.

Le stress oxydatif cutané se traduit par la production de diverses molécules comme les diènes conjugués, générés à partir d'acides gras polyinsaturés, le malonaldéhyde et le tumidine glycol provenant de l'attaque oxydative du génome. Les antioxydants naturels, tel que le tocophérol, ou synthétiques, tel le tocol, permettent de lutter contre les effets délétères des radicaux libres et des peroxydes sur les édifices supramoléculaires cutanés.

La Demanderesse a démontré que, dans des conditions d'oxydation douces, l'application directe de ces antioxydants sur la peau provoque cependant des effets pro-inflammatoires qui sont la conséquence d'une suractivité aux concentrations couramment utilisées.

Les compositions de l'invention permettent avantageusement de remédier à cet effet pro-inflammatoire en fournissant un précurseur d'actif, dont l'activité biologique est très faible voire nulle, et qui est lentement hydrolysé par la glucocérébrosidase pour générer l'antioxydant actif après application de la composition sur la peau. La libération lente de l'actif assure une meilleure biodisponibilité de l'actif dans le milieu cutané et une protection plus efficace.

La présente invention décrit également l'utilisation des compositions contenant un composé apte à libérer le delta-tocophérol, le tocol ou un flavonoïde, pour fabriquer un médicament destiné au traitement de certaines maladies de la peau, notamment la dermite atopique, l'acné ou le psoriasis.

La présente invention a pour objet l'utilisation cosmétique telle que définie à la revendication 2, ou pour la préparation d'un médicament pour application cutanée destiné à traiter une maladie de la peau telle que définie à la revendication 1, d'un composé conjugué de tocol ou de delta-tocophérol avec un glucide.

Les précurseurs d'actif ou composés conjugués selon l'invention peuvent être obtenus par un procédé biochimique ou par synthèse organique. Selon un procédé de synthèse organique, l'actif est couplé au glucide, préalablement tétra-acylé et activé en position 1 (carbone épimérique) par un imidate. Les groupements acétyles sont ensuite hydrolysés par les ions méthanolates.

Les compositions selon l'invention contiennent de 0,001 à 10 % en poids, de préférence 0,01 % en poids, de précurseur d'actif par rapport au poids total de la composition.

La composition selon l'invention peut se présenter sous forme d'émulsion huile dans eau (H/E) ou eau dans huile (E/H). Elle peut encore se présenter sous forme de sphérules comme les liposomes, les nanocapsules ou les nanosphères.

Lorsque la composition est une émulsion, la proportion de la phase grasse va de 5 à 80 % en poids, de préférence de 5 à 50 % en poids, par rapport au poids total de la composition. Les huiles, les émulsionnants et les coémulsionnants utilisés dans la

Lorsque la composition est une émulsion, la proportion de la phase grasse va de 5 à 80 % en poids, de préférence de 5 à 50 % en poids, par rapport au poids total de la composition. Les huiles, les émulsionnants et les coémulsionnants utilisés dans la composition, sous forme d'émulsion, sont choisis parmi ceux classiquement utilisés en cosmétique. L'émulsionnant et le coémulsionnant sont présents, dans la composition, en une proportion allant de 0,3 à 10 % en poids, par rapport au poids total de la composition.

La composition selon l'invention peut également contenir des additifs cosmétiques ou dermatologiques acceptables. Ces additifs peuvent être, en particulier, des tensioactifs, des corps gras, des agents hydratants, des conservateurs, des parfums, des gélifiants, des chélateurs, des pigments comme le Ti02, des filtres et des vitamines libres comme l'acide ascorbique.

La présente invention sera illustrée de façon non limitative par les exemples suivants.

EXEMPLE 1 COMPARATIF: Mise en évidence de l'effet pro-inflammatoire d'un actif non conjugué dans une crème riche en acide gras poly-insaturé de l'art antérieur.

On mesure la concentration en malonaldéhyde (MDA) libéré dans une crème contenant des quantités croissantes d'un agent antioxydant, la vitamine E (ou alpha-tocophérol), que l'on soumet à un stress oxydatif. Rappelons que la quantité de MDA libéré, bon indicateur de l'activité des radicaux libres, inversement proportionnelle à l'efficacité d'un actif antioxydant.

La crème contenant la vitamine E contient également de l'huile de bourrache naturellement riche en acide linoléique. Le stress oxydatif consiste en une exposition solaire de deux heures.

La concentration en MDA libéré est mesurée par complexation avec l'acide thiobarbiturique en milieu acide. Le complexe chromogénique formé absorbe à 532 nm. Sa quantification est suivie par fluorométrie.

Le tableau suivant montre que plus la concentration en vitamine E augmente, plus le MDA est libéré en quantité importante. De toute évidence, la vitamine E engendre un effet pro-inflammatoire dans une crème riche en acide gras poly-insaturé.

| Concentration en vitamine E dans la crème (% massique) | Concentration en MDA libéré (µg MDA/100 mg crème) |
|---|---|
| 0 | 0,7 ± 0.06 |
| 0,05 | 1.2 ± 0.15 |
| 0,1 | 1,9 ± 0,20 |
| 0,15 | 2.0 ± 0.20 |

EXEMPLE 2 : Mise en évidence de la très faible activité des gluco-conjugués de l'invention comparée à celle des actifs libres.

On mesure l'activité antioxydante de divers composés contre la peroxydation d'un lipide insaturé, le squalène. L'indicateur d'activité choisi est le MDA comme dans l'exemple 1.

Le tableau ci-dessous résume les résultats obtenus.

| Composé | Quantité de MDA libéré (µg MDA) |
|---|---|
| Tocol | 6 ± 1,9 |
| Delta-tocophérol | 7,2 ± 1,7 |
| Palmitate de vitamine E | 182,7 ± 39 |
| Delta-tocophéryl-glucopyranoside | 128 ± 38 |
| Tocol-glucopyranoside | 142 ± 28 |

Les précurseurs d'actifs, comme le delta-tocophéryl-glucopyranoside ou le tocol-glucopyranoside possèdent un pouvoir antioxydant faible comparable à celui des esters de vitamine E, comme le palmitate de vitamine E. Par contre, une fois hydrolysée respectivement en delta-tocophérol et en tocol, l'activité antioxydante est révélée.

### EXEMPLE 3 : Stabilité des précurseurs d'actifs selon l'invention

On compare la photostabilité du delta-tocophéryl-glucopyranoside et celle du delta-tocophérol.

Des prises d'essai d'une crème contenant 0,4 % de delta-tocophéryl-glucopyranoside ou de delta-tocophérol sont irradiées par une lumière incidente du 28 J/cm² de 290 à 800 nm (5 DEM).

Le tableau suivant montre que les précurseurs d'actif glucosylés sont au moins deux fois plus photostables que les actifs qu'ils peuvent libérer.

| | | |
|---|---|---|
| Composé | Micromoles | |
| Delta-tocophérol | Irradié | 88 ± 15 |
| | Non irradié | 310 ± 14 |
| Delta-tocophéryl-glucopyranoside | Irradié | 208 ± 4 |
| | Non irradié | 311 ± 7 |

EXEMPLE 4: Cinétique d'hydrolyse de précurseurs glucosylés par la glucocérébrosidase.

Dans un milieu liquide tamponné par un tampon phtalate à pH 5,6, on incorpore le précurseur et l'échantillon du stratum corneum sous forme de poudre. On laisse incuber le tout pendant 24 heures et on réalise un dosage de la quantité d'actif libéré par densitométrie sur une plaque de chromatographie couche mince.

Le tableau suivant donne, pour divers précurseurs glucosylés, la quantité de molécule libérée suite à leur hydrolyse par la glucocérébrosidase en 24 heures par un extrait cutané lyophilisé obtenu après prélèvement par trois strippings successifs de 15 cm².

| Précurseurs glucosylés | Molécule libérée | Quantité de molécule libérée (n moles/heure) |
|---|---|---|
| 4-Méthylumbelliféryl-glucopyranoside | 4-Méthylumbelliférone (substrat de référence) | 100 ± 7 |
| α-Tocophéryl-glucopyranoside | α-Tocophérol | 0 |
| γ-Tocophéryl-glucopyranoside | γ-Tocophérol | 0 |
| δ-Tocophéryl-glucopyranoside | δ-Tocophérol | 14,3 ± 1 |
| Tocol-glucose | Tocol | 14,8 ± 1,2 |
| Phénylglucopyranoside | Phénol | 0,2 ± 0,5 |

En ce qui concerne les cinétiques de libération des dérivés δ-tocophérol et tocol, elles sont 7 fois plus faibles que celles du dérivé de référence (4-méthylumbelliféryl glucopyranoside) utilisé pour le dosage de l'activité de la β-glucocérébrosidase et permet donc d'obtenir des effets dans le temps.

On observe que le α-tocophérol et le γ-tocophérol ne sont pas libérés et demeurent à l'état de précurseur du fait de leur encombrement au voisinage de la liaison C-1.

Dans les exemples suivants, les proportions sont en pourcentage massique:

### EXEMPLE 5 :

| | |
|---|---|
| Delta-tocophéryl béta-dl-glucopyranoside ou tocol-béta-dl-glucopyranoside | 0,01 |
| Huile de vaseline. | 20 |
| Squalane | 5 |
| Palmitate d'octyle | 20 |
| PEG-40 stéarate | 2 |
| Conservateur | 0,2 |
| Parfum | 0,15 |
| Glycérine | 5 |
| Eau QSP | 100 |

### EXEMPLE 6:

| | |
|---|---|
| Delta-tocophéryl-béta-dl-glucopyranoside Ou tocol-bêta-dl-glucopyranoside | 0,01 |
| Huile naturelle de bourrache | 20 |
| Huile de vaseline | 10 |
| PEG-40 stéarate | 4 |
| Conservateur | 0,2 |
| Parfum | 0,15 |
| Glycérine | 10 |
| Eau QSP | 100 |

## Revendications

1. Utilisation d'un composé conjugué d'une substance biologiquement active avec un glucide tel que :
- ladite substance biologiquement active est choisie parmi le tocol ou le delta-tocophérol, et
- la liaison chimique entre ladite substance biologiquement active et ledit glucide est une fonction éther entre le glucide et la fonction phénol de la substance biologiquement active,
pour la préparation d'un médicament pour application cutanée destiné à traiter une maladie de la peau.

2. Utilisation selon la revendication 1, **caractérisée en ce que** la maladie de la peau est une dermite atopique, l'acné ou le psoriasis.

3. Utilisation d'une composition cosmétique pour application cutanée comprenant un composé conjugué d'une substance biologiquement active avec un glucide tel que :
- ladite substance biologiquement active est choisie parmi le tocol ou le delta-tocophérol, et
- la liaison chimique entre ladite substance biologiquement active et ledit glucide est une fonction éther entre le glucide et la fonction phénol de la substance biologiquement active, contre le vieillissement cutané, contre le dessèchement de la peau, pour apaiser les effets dus aux expositions solaires et polluantes, ou contre la production de radicaux libres.

4. Composé conjugué du tocol avec un glucide tel que la liaison chimique entre le tocol et ledit glucide est une fonction éther entre le glucide et la fonction phénol du tocol.

5. Composé selon la revendication 4, **caractérisé en ce que** le glucide est un ose en C3-C6, notamment le glucose.

6. Composition cosmétique ou pharmaceutique pour application cutanée contenant le composé conjugué selon les revendications 4 ou 5.

7. Composition selon la revendication 6, **caractérisée en ce qu'**elle contient 0,001 à 10 % en poids du composé conjugué, par rapport au poids total de la composition.

## Claims

1. Use of a conjugated compound of a biologically active substance with a carbohydrate such that:
- said biologically active substance is chosen from tocol or delta-tocopherol, and
- the chemical bond between said biologically active substance and said carbohydrate is an ether functional group between the carbohydrate and the phenol functional group of the biologically active substance,
for the preparation of a medicament for skin application, intended to treat a skin disease.

2. Use according to Claim 1, **characterized in that** the skin disease is atopic dermatitis, acne or psoriasis.

3. Use of a cosmetic composition for skin application, comprising a conjugated compound of a biologically active substance with a carbohydrate such that:
- said biologically active substance is chosen from tocol or delta-tocopherol, and
- the chemical bond between said biologically active substance and said carbohydrate is an ether functional group between the carbohydrate and the phenol functional group of the biologically active substance, against skin ageing, against drying of the skin, for soothing the effects due to exposure to solar radiation and to pollutants, or against the production of free radicals.

4. Conjugated compound of tocol with a carbohydrate such that the chemical bond between the tocol and said carbohydrate is an ether functional group between the carbohydrate and the phenol functional group of the tocol.

5. Compound according to Claim 4, **characterized in that** the carbohydrate is a C3-C6 monosaccharide, in particular glucose.

6. Cosmetic or pharmaceutical composition for skin application containing the conjugated compound according to Claim 4 or 5.

7. Composition according to Claim 6, **characterized in that** it contains 0.001 to 10% by weight of the conjugated compound, relative to the total weight of the composition.

## Patentansprüche

1. Verwendung einer konjugierten Verbindung einer biologisch aktiven Substanz mit einem Kohlenhydrat, derart, dass:
- die biologisch aktive Substanz aus Tocol und delta-Tocopherol ausgewählt ist und
- die chemische Bindung zwischen der biologisch aktiven Substanz und dem Kohlenhydrat eine Ether-Funktion zwischen dem Kohlenhydrat und der Phenol-Funktion der biologisch aktiven Substanz ist,
für die Herstellung eines Medikaments für die kutane Anwendung, das dazu bestimmt ist, eine Hautkrankheit zu behandeln.

2. Verwendung nach Anspruch 1, **dadurch gekennzeichnet, dass** die Hautkrankheit eine atopische Dermatitis, Akne oder Psoriasis ist.

3. Verwendung einer kosmetischen Zusammensetzung für die kutane Anwendung, umfassend eine konjugierte Verbindung einer biologisch aktiven Substanz mit einem Kohlenhydrat, derart, dass:
- die biologisch aktive Substanz aus Tocol und delta-Tocopherol ausgewählt ist und
- die chemische Bindung zwischen der biologisch aktiven Substanz und dem Kohlenhydrat eine Ether-Funktion zwischen dem Kohlenhydrat und der Phenol-Funktion der biologisch aktiven Substanz ist,
gegen Hautalterung, gegen Austrocknen der Haut, um die Auswirkungen aufgrund von Sonnen- und Umweltverschmutzungseinwirkung zu lindern oder gegen die Produktion von freien Radikalen.

4. Konjugierte Verbindung von Tocol mit einem Kohlenhydrat, derart, dass die chemische Bindung zwischen dem Tocol und dem Kohlenhydrat eine Ether-Funktion zwischen dem Kohlenhydrat und der Phenol-Funktion von Tocol ist.

5. Verbindung nach Anspruch 4, **dadurch gekennzeichnet, dass** das Kohlenhydrat eine (C3-C6)-Ose, insbesondere Glucose, ist.

6. Kosmetische oder pharmazeutische Zusammensetzung für die kutane Anwendung, welche die konjugierte Verbindung nach den Ansprüchen 4 oder 5 enthält.

7. Zusammensetzung nach Anspruch 6, **dadurch gekennzeichnet, dass** sie 0,001 bis 10 Gew.-% der konjugierten Verbindung, bezogen auf das Gesamtgewicht der Zusammensetzung, enthält.
